# EUROPEAN PATENT APPLICATION

(11) **EP 2 263 628 A1**
(43) Date of publication of application: **22.12.2010**
(21) Application number: 10011143.4
(22) Date of filing: 16.01.2003
(51) Int. Cl.: A61F 13/20, D01F 2/00, D06M 11/05

(54) **High absorbency lyocell fibers and method for producing same**

(30) Priority: 24.01.2002 US 57352
(62) Divisional of application: 03703866.8
(71) Applicant: McNeil-PPC, Inc., Skillman, NJ 08558 (US)
(72) Inventor: Nguyen, Hien V., East Windsor, NJ 08520 (US)
(74) Representative: Metten, Karl-Heinz

(57) **Abstract**

High absorbency lyocell fibers are obtainable by hydrothermal treatment. The fibers can be treated with water at temperatures of at least about 60° C to provice lyocell fibers that can be formed into a random fibrous plug having a mass of 2 g, a density of 4 g/cm³, and a diameter of 25 mm which has a GAT Absorbency (at 15 min.) of at least about 3.7 g/g.

## Description

### Field of the Invention

This invention relates to a method for improving the absorbency characteristics of lyocell fibers, and more particularly to such a method which is useful in the preparation of absorbent materials for catamenial tampons, sanitary napkins, and other absorbent dressings.

### Background of the Invention

There are several forms of rayon manufactured and used in various industries including viscose, cuprammonium, high-wet modulus and lyocell. However, lyocell is distinct from other rayons. For example, the degree of crystallinity of lyocell is at least twice that of rayon; lyocell consists of rather well defined fibrils which can separate due to wet abrasive action, but it is otherwise not very sensitive to water; and has higher tensile strength, especially wet tensile strength compared to other regenerated cellulose fibers. Thus, while viscose rayon has seen widespread use in absorbent articles, such as catamenial tampons, sanitary napkins, and other absorbent dressings, lyocell has not been used in any significant quantities for these articles.

Various techniques have been described in the literature for increasing the absorbency of cellulosic materials. Such techniques include, for example, the preparation of alloy fibers having matrices of regenerated cellulose and, uniformly dispersed therein, polyacrylates (e.g., Smith US Pat. No. 3,884,287), acrylate/methacrylate copolymers (e.g., Allen et al U..S. Pat. No. 4,066,584; Meierhoefer US Pat. No. 4,104,214; and Allen US Pat. No. 4,240,937), alkylene vinyl ether/ethylene dicarboxylic acid copolymers (e.g., Denning US Pat. No. 4,165,743), sulfonic acids (e.g., Allen US Pat. No. 4,242,242, polyvinylpyrrolidone (e.g., Smith US Pat. No. 4,136,697), cellulose sulfate (e.g., Smith US Pat. No. 4,273,118), carboxymethylcellulose (e.g., Smith U. S. Pat. No. 4,289,824), or the like.

Viscose rayon or other regenerated cellulose polymer alloy fibers may be subjected during preparation to one or more hot, aqueous baths. For example, post-regeneration treatments in hot water baths at temperatures of from ambient (20°-25° C) to as much as 100° C. have been described in various of the above patents. Such treatments have not, however, been disclosed as having any appreciable effects on the absorbency characteristics of the cellulosic materials.

Other treatments of cellulosic fibrous materials have been proposed in the literature for increasing their absorbency. Thus, the treatment of carboxymethylcellulose fibers in hot aqueous baths containing cross-linking agents has been proposed to effect wet cross-linking of the fibers, with consequent increase in the absorbency thereof (see, for example, Steiger US Pat. No. 3,241,553; Ells US Pat. No. 3,618,607; and Chatterjee US Pat. No. 3,971,379). None of this literature, however, suggests the use of hot water treatments per se for improving the absorbency of the cellulosic materials thereof.

High Temperature Water (preferably deionized) Treatment of cellulosic fibrous materials has been described in Shah et al., US Pat. No. 4,575,376. This treatment is at 95°-100° C, and materials actually subjected to this treatment appear to be limited to cotton, viscose rayon, and viscose rayon alloys. Due to the different water absorbency characteristics of viscose rayon from lyocell, the data provided in this reference does not necessarily suggest that the absorbency of lyocell would be improved as significantly as other cellulosic fibers in such a HTWT process.

Tyler et al., US Pat. No. 4,919,681, purports to disclose a modified method of treating cellulose fibers in an acid solution having a pH of no more than 4. Again, the cellulosic fibers actually tested in this reference are limited, and the data does not necessarily suggest that the absorbency of lyocell would be improved as significantly as other cellulosic fibers in such a process.

### Summary Of The Invention

The present invention provides high absorbency lyocell fibers to allow their use as a replacement for conventional viscose rayon fibers in absorbent articles.

In addition, it provides a new technique for treating lyocell fibrous materials to increase their absorbency.

This result is obtained in accordance herewith by a hydrothermal treatment, which comprises heating the lyocell fibers in the presence of water at temperatures within the range of up to about 100° C, for a period sufficient to increase the absorbency of the fibers to provide a Syngyna tampon absorbency of at least about 4.4 g/g (at a density of about 0.4 g/cm³) and drying the treated fibers.

### Brief Description Of The Drawing

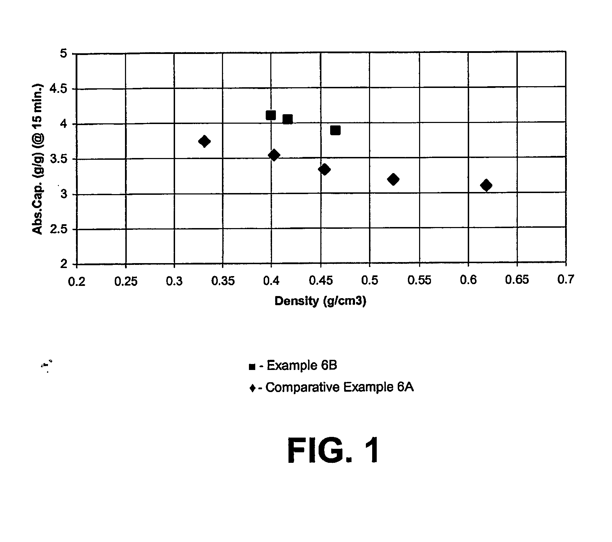

Fig. 1 is a graph of the absorbent capacity of the fibrous plugs of Example 6B of the present invention and Comparative Example 6A as a function of their density.

### Description Of The Preferred Embodiments

Hydrothermal treatment of lyocell fibers desirably occurs in a water bath at a temperature of from about 60° C to 100° C (or greater if under pressure). This treatment provides lyocell fibers that exhibit high absorbency in compressed structures that is similar to the levels of absorbency provided by viscose rayon. The fibers are treated for a sufficient period to increase their absorbency as measured by the Syngyna Test, as described hereinbelow. It has been found that the absorbency of the lyocell fibers has been increased by at least 14% and as much as 30% when treated at 90° C to 100° C.

Hydrothermal treatment temperature can be from room temperature up to above boiling point, 100° C (of course under pressure greater than atmospheric pressure or with added salt to raise the normal boiling point). With lower temperatures, such as room temperature, the residence time needs to be higher than high temperature conditions.

The processing steps of the invention are carried out in water baths. It is not believed that it is necessary to use deionized water to achieve high absorbency lyocell fibers. For example, it is believed that ionic materials in the water such as sodium, chloride, sulfate atec may be present, without greatly affecting the treatment. At the present time, it is believed that low pH, i.e. acidic condition, should be avoided, neutral to high pH are acceptable.

When it is desired to utilize the thus treated fibers as absorbent materials for catamenial tampons or sanitary napkins they may thereafter be dried, compressed, formed into webs (as, for example, by carding or air-forming), and then formed into absorbent articles. When, for example, it is desired to produce tampons therefrom, webs of the hydrothermally treated lyocell fibers may be formed into tampons by the procedure described in Friese et al., US Pat. No. 6,310,269 (the disclosure of which is herein incorporated by reference), owned by the assignee of the present invention.

The hydrothermally treated lyocell fibers may be combined with other materials to form the absorbent structure used in the absorbent articles, described above. For example, the materials employed in the formation of an absorbent article according to the present invention include the treated lyocell fibers, additional fibers, foam, hydrogels, wood pulp, superabsorbents, and the like. A useful, non-limiting list of useful fibers includes natural fibers such as cotton, wood pulp, jute, and the like; and processed fibers such as regenerated cellulose, cellulose nitrate, cellulose acetate, rayon (other than treated lyocell), polyester, polyvinyl alcohol, polyolefin, polyamine, polyamide, polyacrylonitrile, and the like. Other fibers in addition to the above fibers may be included to add desirable characteristics to the absorbent body.

Fibers useful in the present invention include absorbent fibers that are capable of absorbing a liquid into the fiber itself and non-absorbent fibers that do not absorb significant amounts of liquid, but which can help to provide a structure which is capable of holding liquids in interfiber capillaries. Absorbent fibers include, without limitation, cotton, wood pulp, jute, regenerated cellulose, cellulose nitrate, cellulose acetate, rayon, and the like. Non-absorbent fibers include, without limitation, polyester, polyolefin, polyamine, polyamide, polyacrylonitrile, and the like.

The water baths (or sprays or the like) used in the method of this invention may also contain various adjuvants to impart other desired properties to the treated fibers. For example, it is preferred in the processing of lyocell fibers to be utilized as absorbents for catamenial tampons to incorporate in the hydrothermal treatment baths finishing agents, lubricating agents or other desirable agents. These may be present as mixtures of the various agents, and other conventional additives may of course also be incorporated in the hydrothermal treatment baths of the invention, as desired. The agents may include diols, surfactants, and finishes such as glycerol monolaurate and similar compounds as disclosed in Brown-Skrobot, US Pat. No. 5,679,369 (the disclosure of which is herein incorporated by reference), owned by the assignee of the present invention.

As used herein, the term "surfactant" refers to a surface active agent, i.e., one that modifies the nature of surfaces. Surfactants are often used as wetting agents, detergents, emulsifiers, dispersing agents, penetrants, and antifoaming agents. Surfactants may be anionic, cationic, nonionic and ampholytic. Preferably, the surfactant used in the present invention is a nonionic surfactant. Nonionic surfactants are generally less irritating of human body tissue, and they are therefore more acceptable in uses that contact such tissue.

A representative, non-limiting list of useful diols includes C₂₋₈ diols and polyglycols, and the like. Preferably, the diol is selected from the group consisting of glycols (C₂ and C₃ diols) and polyglycols.

As used in the specification and the claims, the term "polyglycol" refers to a dihydroxy ether formed by dehydration of two or more glycol molecules. A representative, non-limiting list of useful polyglycols includes ethylene glycol, propylene glycol, polyethylene glycols, plypropylene glycols, methoxypolyethylene glycols, polybutylene glycols, or block copolymers of butylene oxide and ethylene oxide.

Preferred nonionic surfactants are ethoxylates, including fatty acid ester ethoxylates, fatty acid ether ethoxylates, and ethoxylated sugar derivatives.

Examples of ethoxylated fatty acid esters can be found in the class of ethoxylated fatty acid polyolesters, and more particularly, ethoxylated fatty acid sorbitan ester. A representative, non-limiting list of useful ethoxylated fatty acid sorbitan esters includes polyoxyethylene sorbitan laurate (also known as Polysorbate 20 and 21), polyoxyethylene sorbitan palmitate (also known as Polysorbate 40), polyoxyethylene sorbitan stearate (also known as Polysorbate 60 and 61), polyoxyethylene sorbitan tristearate (also known as Polysorbate 65), polyoxyethylene sorbitan oleate (also known as Polysorbate 80 and 81), and polyoxyethylene sorbitan trioleate (also known as Polysorbate 85).

Examples of ethoxylated fatty acid ethers can be found in the class of polyoxyethylene alkyl ether. A representative, non-limiting list of useful polyoxyethylene alkyl ethers includes polyoxyethylene lauryl ether, polyoxyethylene stearyl ether (also known as Steareth-2, Steareth-10, and the like), polyoxyethylene cetyl ether (also known as Ceteth-2, Ceteth-10, and the like), and polyoxyethylene oleyl ether (also known as Oleth-2, Oleth-10, and the like).

Examples of fatty acid esters can be found in the class of sorbitan fatty acid esters. A representative, non-limiting list of useful sorbitan fatty acid esters includes sorbitan monooleate, sorbitan monostearate, sorbitan monopalmitate, sorbitan monolaurate, sorbitan tristearate, and sorbitan trioleate.

Examples of ethoxylated sugar derivatives can be found in the class of methyl glucose derivatives. A representative, non-limiting list of useful methyl glucose derivatives includes methyl gluceth-10, methyl glucose-20, methyl glucose-20 distearate, methyl glucose dioleate, and methyl glucose sesquistearate, PEG-120 methyl glucose dioleate, and PEG-20 methyl glucose sesquistearate.

Examples of pharmaceutically active compounds includes those such as Glycerol Monolaurate ("GML", useful to inhibit the production of toxic shock syndrome toxin-1 during the use of tampons) such as are disclosed in Brown-Skrobot, US Patent number 5,679,369, the disclosure of which is herein incorporated by reference.

Desirably, such agents or other additives are incorporated in minor amounts in the hydrothermal treatment baths in order that the amounts of such materials deposited on the lyocell fibers are well below 5 wt-%, and preferably within the range of from about 0.1 to 1 wt-%.

The improved absorbencies achieved by the present method may be determined in vitro, or in vivo in appropriately conducted clinical evaluations. For example, either the well-known Syngyna Test (see the US Federal Register, Part III, Department of Health and Human Services, Food and Drug Administration (21 CFR §801.430, April 1, 2001)), or the Plug Test (see Examples 5 and 6, below), may be utilized for the in vitro measurement of the absorbencies exhibited by the hygrothremally treated lyocell fibers.

The highly absorbent lyocell fibers of the present invention can be formed into an absorbent tampon having a Syngyna Absorbency of at least about 4.4 g/g and preferably, at least about 4.8 g/g. Alternatively, the highly absorbent lyocell fibers can be classified by forming a mass of 2 g into a plug (as described for Examples 5 and 6, below) having a density of 0.4 g/g and a diameter of 25 mm and a GAT Absorbency (at 15 min.) of at least about 3.7 g/g, preferably at least about 4 g/g.

### Examples

The techniques utilized in the hydrothermal treatment of the invention, and the improved absorbency characteristics thus obtained, are illustrated in the following examples, wherein all temperatures are given in degrees Celsius and all parts and percentages are by weight, unless otherwise indicated.

### Examples 1-4

Laboratory-made tampons having a mass of about 2.5 g were made according to the general teaching of Friese et al., US Pat. No. 6,310,296, the disclosure of which is hereby incorporated by reference. The tampons were then subjected to the Syngyna Test as described in the US Federal Register, Part III, Department of Health and Human Services, Food and Drug Administration (21 CFR §801.430, April 1, 2001). The results are shown in Table 1, below:

**Table 1**

| Sample | Description | Density (g/cm³) | Syngyna Value for tampon (g) | Syngyna Value (g/g) |
|---|---|---|---|---|
| Comparative Example 1A | 1.5 denier TENCEL^{®} | 0.389 | 9.92 | 3.89 |
| Example 1B | Treated¹ 1.5 denier TENCEL^{®} | 0.390 | 11.37 | 4.44 |
| Comparative Example 2A | 2.2 denier TENCEL^{®} | 0.394 | 10.32 | 3.97 |
| Example 2B | Treated¹ 2.2 denier TENCEL^{®} | 0.319 | 10.96 | 5.18 |
| Comparative Example 3A | 3 denier TENCEL^{®} | 0.406 | 10.56 | 4.14 |
| Example 3B | Treated¹ 3 denier TENCEL^{®} | 0.406 | 12.36 | 4.82 |
| Comparative Example 4A | 1.5 denier TENCEL^{®} | 0.39 | 9.97 | 3.86 |
| Example 4B | Treated² 1.5 denier TENCEL^{®} | 0.38 | 12.39 | 4.80 |

| | | | | |
|---|---|---|---|---|
| ¹ Hydrothermal treatment in boiling water at 90-100° C for about 1 hour. ² Hydrothermal treatment in boiling water at about 125° C for about 1 hour at elevated pressure. | | | | |

The Syngyna Test data illustrate that hydrothermal treatment of lyocell fibers improves their absorbency in compressed tampons to over 4.4 g/g. This improvement represents an increase of 14% to 30%.

### Examples 5 and 6

Compressed fibrous structures can take advantage of the present invention. The improvements can be shown by use of the Plug Test described hereinbelow.

### Plug Test

The fiber blend is opened via standard fiber opening and carding equipment.

Sample Preparation Procedure: A fixed amount of fiber blend, of weight 2 grams, is introduced into a stainless steel mold with a cylindrical cavity (of diameter 1 inch). A cylindrical plunger which fits into the cavity is used to compress the fiber mass. A laboratory press is used to apply the necessary pressure.

After coming out of the mold the plug is left on the bench for about 20-30 minutes to equilibrate to an equilibrated thickness, from which the density of the plug is calculated.

Plug Absorbency Test: The test sample is the equilibrated plug. The plug is tested in a Gravimetric Absorbency Tester (as described in Pronoy K. Chatterjee and Hien V. Nguyen, "Mechanism of Liquid Flow and Structure Property Relationships", Chapter II, Absorbency, Textile Science and Technology, Vol. 7, pp 29-84, at pp. 67-68 (Elsevier Science Publishers B.V.), the disclosure of which is herein incorporated by reference) using 1% saline as test fluid. The test cell is a multi-hole cell having 25 holes, each having a diameter of about 3 mm, arranged in a circular array having a diameter of about 37 mm with two rings about a central hole (eight holes in the first ring and 16 holes in the outer ring) with a GF/A filter paper on top. The test is carried out at 1 cm hydrostatic head: the filter paper is 1 cm higher than the fluid level in the reservoir. A hollow cylinder of diameter slightly greater than the mold cavity is placed vertically on the filter paper. At the start of the test the fiber plug is dropped into the cylinder and a weight is placed on top to impose an equivalence of 0.5-psi pressure.

The amount absorbed is recorded with time by a computer. The test takes about 10 minutes.

**Table 2**

| Sample | Description | Density (g/cm³) | GAT Absorbency @ 45 sec. (g/g) | GAT Absorbency @ 15 min. (g/g) |
|---|---|---|---|---|
| Comparative Example 5A | 1.5 denier TENCEL^{®} | 0.40 | 2.84 | 3.22 |
| Example 5B | Treated¹ 1.5 denier TENCEL^{®} | 0.38 | 2.84 | 4.11 |
| Comparative Example 6A | 2.2 denier TENCEL^{®} | See the graph in Fig. 1 | | |
| Example 6B | Treated¹ 2.2 denier TENCEL^{®} | | | |

| | | | | |
|---|---|---|---|---|
| ¹ Hydrothermal treatment in boiling water at 90-100° C for about 1 hour. | | | | |

The Plug Test data illustrate that hydrothermal treatment of lyocell fibers significantly improves their GAT absorbency (@ 15 min.) in compressed plugs, e.g., to over 3.7 g/g at a density of 0.4 g/cm³.

The specification and embodiments above are presented to aid in the complete and non-limiting understanding of the invention disclosed herein. Since many variations and embodiments of the invention can be made without departing from its spirit and scope, the invention resides in the claims hereinafter appended.

## Claims

1. A fibrous structure comprising lyocell fibers capable of being formed into a random fibrous plug having a mass of 2 g, a density of 4 g/cm³, and a diameter of 25 mm which has a GAT Absorbency (at 15 min.) of at least about 3.7 g/g.

2. The fibrous structure of claim 1 wherein the lyocell fibers are capable of being formed into a random fibrous plug having a mass of 2 g, a density of 4 g/cm³ , and a diameter of 25 mm which has a GAT Absorbency (at 15 min.) of at least about 4 g/g.

3. The fibrous structure of claim 1 which further comprises additional fibers.

4. The fibrous structure of claim 3 wherein the additional fibers comprise absorbent fibers.

5. The fibrous structure of claim 3 wherein the additional fibrous material comprises non-absorbent fibers.

6. The fibrous structure of claim 1 which further comprises additional materials.

7. The fibrous structure of claim 6 wherein the additional materials comprise materials selected from the group consisting of foam, hydrogel, superabsorbent, and combinations thereof.
